Europäisches Patentamt

European Patent Office

Office européen des brevets

(19)

(11) Veröffentlichungsnummer: **0 005 738**
**B1**

(12) **EUROPÄISCHE PATENTSCHRIFT**

(21) Anmeldenummer: **79101323.8**

(22) Anmeldetag: **02.05.79**

(51) Int. Cl.³: **C 07 D 307/89, C 07 B 1/00**

(54) Verfahren zur Herstellung von Hexahydrophthalsäure anhydrid

(30) Priorität: **26.05.78 DE 2823164**

(43) Veröffentlichungstag der Anmeldung:
**12.12.79 Patentblatt 79/25**

(45) Bekanntmachung des Hinweises auf die Patenterteilung:
**26.11.80 Patentblatt 80/24**

(84) Benannte Vertragsstaaten:
**BE CH DE FR GB IT NL**

(56) Entgegenhaltung:
**DE - C - 961 622**

(73) Patentinhaber: **BAYER AG**
**Zentralbereich Patente, Marken und Lizenzen**
**D - 5090 Leverkusen 1**
**Bayerwerk (DE)**

(72) Erfinder: **Halcour, Kurt, Dr.**
**Gellertstrasse 14**
**D - 5090 Leverkusen (DE)**
**Waldmann, Helmut, Dr.**
**Carl-Rumpff-Strasse 59**
**D - 5090 Leverkusen (DE)**
**Schwerdtel, Wulf, Dr.**
**Hegelstrasse 9**
**D - 5090 Leverkusen (DE)**

Courier Press, Leamington Spa, England.

## Verfahren zur Herstellung von Hexahydrophthalsäure-anhydrid

Die vorliegende Erfindung betrifft ein Verfahren zur Hydrierung von Δ4-Tetrahydrophthalsäureanhydrid zu Hexahydrophthalsäureanhydrid ohne Verwendung eines Fremdlösungsmittels in der Riesel- oder Flutphase über einen im Festbett angeordneten Katalysator aus Palladium und/oder Ruthenium und/oder Nickel, der auf einem Träger aus Aluminiumoxid, das mindestens zu 20% in Lithium-Aluminium-Spinell umgewandelt wurde, aufgebracht, ist.

Es ist bekannt, Δ4-Tetrahydrophthalsäureanhydrid zu Hexahydrophthalsäureanhydrid zu hydrieren.

Dazu wird das zu hydrierende Tetrahydrophthalsäureanhydrid in den meisten Fällen in einem Fremdösungsmittel gelöst.

Beispielsweise wird gemäß US—PS 2 601 705 Tetrahydrophthalsäureanhydrid in Äthylacetat und gemäß J. Amer. chem. Soc. vol. 60 (1938) p. 2145 in Eisessig gelöst, der Hydrierungskatalysator, Raney-Nickel bzw. fein verteiltes Platinschwarz (nach Adam), suspendiert und hydriert. Ein Nachteil dieser Verfahren besteht daran, daß das Fremdlösungsmittel und der Katalysator aus dem Hydriergut entfernt werden müssen. Ein anderer Nachteil liegt in den zu langen Hydrierzeiten.

Aus der DE—PS 961 622 ist es bekannt, Δ4-Tetrahydrophthalsäureanhydrid in der Schmelze in Gegenwart von suspendiertem, auf Kieselsäure niedergeschlagenem Nickelkatalysator zu Hexahydrophthalsäureanhydrid zu hydrieren. Auch hier muß der Katalysator durch Filtration abgetrennt werden, oder aber es sind lange Absetzzeiten erforderlich, nach denen dann das Hydrierungsprodukt vom abgesetzten Katalysator getrennt werden kann. Außerdem sind relativ lange Hydrierzeiten notwendig.

Gemäß DE—PS 1 226 556 sollen ungesättigte Carbonsäureanhydride, wie beispeilsweise Maleinsäureanhydrid oder Tetrahydrophthalsäureanhydrid in flüssiger Phase, d.h. unter Verwendung von Fremdlösungsmitteln. oder in der Schmelze, durch katalytische Hydrierung mittels im Festbett angeordneter Kupfer-, Kobalt- oder Nickelmolybdate, -wolframate, -chromate oder -vanadate nach dem Rieselverfahren kontinuierlich in die entsprechendengesättigten Carbonsäureanhydride überführt werden können. Im Falle des Tetrahydrophthalsäureanhydrid wird die Hydrierung jedoch in dem Fremdlösungsmittel Dioxan vorgenommen. Nachteilig bei diesem Verfahren ist auch der erforderliche hohe Katalysatorgehalt, der nach den Beispielen dieser Schrift etwa 10—20 Gew.-% -bezogen auf imprägniertes Trägermaterial — beträgt.

Aufgabe der vorliegenden Erfindung war es, Δ4-Tetrahydrophthalsäureanhydrid praktisch qauntitativ und in hohen Raum-Zeit-Ausbeuten kontinuierlich zu Hexahydrophthalsäureanhydrid zu hydrieren. Das Verfahren sollte wirtschaftlich arbeiten und die geschilderten Nachteile der Verfahren des Standes der Technik vermeiden.

Die Aufgabe wurde dadurch gelöst, daß die Hydrierung in der Riesel- und Flutphase erfolgt unter Verwendung spezifischer Katalysatoren auf speziellen Trägern im Festbett und unter Ausnutzung des hydrierten Produktes als Lösungsmittels.

Gegenstand der Erfindung is somit ein Verfahren zur katalytischen, kontinuierlichen Hydrierung von Δ4-Tetrahydrophthalsäureanhydrid zu Hexahydrophthalsäureanhydrid in Gegenwart mindestens eines im Festbett angeordneten Hydrierungskatalysators in flüssiger Phase bei Temperaturen von 70—150°C und 30—200 bar in der Riesel- oder Flutphase, dadurch gekennzeichnet, daß man die Hydrierung in Gegenwart eines im Festbett angeordneten Katalysators aus der Reihe Palladium, Ruthenium oder Nickel oder deren Kombinationen durchführt, der Katalysator auf einem Träger aus Aluminiumoxid, das mindestens zu 20% in Lithium - Aluminium - Spinell umgewandelt worden ist, aufgebracht ist, und die zu hydrierende flüssige Phase aus einem Gemisch von Tetrahydrophthalsäureanhydrid und Hexahydrophthalsäureanhydrid im Gewichtsverhältnis 1:1 bis 1:100 besteht.

Das als Ausgangsmaterial dienende Tetrahydrophthalsäureanhydrid kann in bekannter Weise durch Synthese aus Butadien und Maleinsäureanhydrid (Diensynthese nach Diels-Alder) erhalten werden, wobei im wesentlichen das cis-Δ4-Isomere entfällt.

Die Hydrierung erfolgt an im Festbett angeordneten Katalysatoren in der Riesel- oder Flutphase in einer geeigneten Hydrierapparatur.

Der im Festbett angeordnete Katalysator kann in einen Schachtofen eingefüllt sein. Wegen der besseren Wärmeabfuhr ist es jedoch vorteilhaft, den Katalysator in einen Rohrbündelreaktor einzufüllen, wobei um die Rohre ein Wärmeabfuhrmedium geleitet wird.

Die Durchführung der Hydrierung kann in der Riesel- oder Flutphase erfolgen. Erflogt die Hydrierung in der Riesel-phase, so rieselt das zu hydrierende Produkt im Reaktor von oben nach unten über den Katalysator, der sich in einer Wasserstoffatmosphäre befindet; der Wasserstof kann von oben oder von unten in den Reaktor eingeführt werden. Einen Teil des Abgases entspannt man nach Abtrennung des flüssigen Produktes ins Freie. Führt man die Hydrierung in der Flutphase durch, so durchströmen Einsatzprodukt und Wasserstoff, meist in gelöster Form, das Katalysatorbett von unten nach oben. Die Hydrierdauer in der Rieselphase ist von der Länge des Reaktors abhängig; sie beträgt bei einer Reaktorlänge von 6 m weniger als 10 Minuten. Bei der Flutphase ist die Hydrierdauer abhängig von der Einsatzmenge un der Reaktorlänge. Sie beträgt aber auch nur höchstens eine Stunde.

Obwohl die Hydrierung von Δ4-Tetrahydrophthalsäureanhydrid prinzipiell in unverdünnter Form, d.h. in der Schmelze erfolgen kann, hat es sich als äußerst vorteilhaft erwiesen, das als Reaktionsprodukt anfallende Hexahydrophthalsäureanhydrid als Verdünnungsmittel einzusetzen, da hierdurch einerseits eine Verringerung der lokalen Wärmetönung erflogt und andererseits der Schmelzpunkt der Einsatzmischung von etwa 102°C auf 40—60°C erniedrigt werden kann. Eine Abtrennung des benutzten Verdünnungsmittels vom Hydrierprodukt ist nicht erforderlich, da es mit dem Hydrierprodukt identisch ist. Man kann einen Teil des Hydrierproduktes direkt wieder zum Verdünnen des Δ4-Tetrahydrophthalsäureanhydrids zurückpumpen. In diesem Fall kann das Gewichtsverhältnis von Hexahydrophthalsäureanhydrid zu eingesetztem 4-Tetrahydrophthalsäureanhydrid 1:1 bis 1:100, vorzugsweise 1:5 bis 1:30 betragen.

Als Hydriergase können Wasserstoff oder Wasserstoffenthaltende Gase verwendet werden, wobei enthaltende Katalysatorgifte vorher zu entfernen sind. Nach der Reaktion kann ein kleiner Anteil des Restgases entspannt werden, um die Anreicherung von Intertgasen zu vermeiden.

Für die Hydrierung von Δ4-Tetrahydrophthalsäureanhydrid zu vorzugsweise cis-Hexahydrophthalsäureanhydrid nach dem erfindungsgemäßen Verfahren sind spezielle Katalysatoren von Vorteil; in besonderem Maße eignen sich Palladium und/oder Ruthenium oder Nickel, vorzugsweise Palladium und/oder Ruthenium, insbesondere Palladium.

Die Katalysatoren sind mit einem Gehalt von 0,1 bis 5 Gew.-% auf Katalysatorträger aufgebracht. Als Katalysatorträger wird Aluminiumoxid eingesetzt, welches zu mindestens 20% in Lithium-Aluminium-Spinell umgewandelt worden ist. Der Katalysatorträger kann als Zylinder, Extrudat, Pellet oder stückig eingesetzt werden, bevorzugt ist jedoch die Kugelform (Durchmesser ca. 1 bis 8 mm).

Die Hydrierung in der flüssigen Phase wird bei Drucken oberhalb von 30 bar durchgeführt; im allgemeinen werden Drucke von 50 bis 200 bar, vorzugsweise von 80—150 bar angewendet.

Die Temperaturen für die Hydrierung von Δ4-Tetrahydrophthalsäureanhydrid sollten 70 bis 150°C betragen; bevorzugt wird ein Temperaturbereich von 90 bis 110°C.

Die Raum-Zeit-Ausbeute kann 0,1 bis 5 kg Hexahydrophthalsäureanhydrid pro Liter Katalysator (= Katalysator + Katalysatorträger) und Stunde betragen, vorzugsweise unter 2 kg/l × h.

Hexahydrophthalsäureanhydrid kann als Härter für 1,2-Polyepoxide und/oder als Zwischenprodukt für die Herstellung von Kunstharzen wie Polyepoxidharze und Polyesterharze eingesetzt werden.

Das erfindungsgemäße Verfahren wird durch die nachfolgenden Beispiele näher erläutert. Die angegebenen Prozentgehalte beziehen sich auf sas Gewicht, sofern nicht anders vermerkt.

### Beschreibung der Hydrierapparatur

Als Reaktor diente ein 4 bzw. 6m langes Rohr mite einer lichten Weite von 24 mm. Das Reaktionsrohr war mit einem Doppelmantel versehen, welches mit Dampf geheizt und mid Kondensat gekühlt werden konnte. Der Katalysator war in dem Reaktionsrohr fest angeordnet. Über den Katalysator wurde im Falle der Rieselphasehydrierung das zu hydrierende Einsatzprodukt, verdünnt mit rückgeführtem Hydrierprodukt, von oben aufgegeben.

Am unteren Ende des Reaktors befand sich ein Abscheider, in dem das flüssige Reaktionsprodukt vom Restgas abgeschieden wurde. Meist wurde ein Teil des Reaktionsproduktes zum Einsatz zurückgepumpt, das restliche Reaktionsprodukt wurde ins Freie entspannt. Der Wasserstoff wurde gemeinsam mit dem flüssigen Einsatzprodukt am oberen Ende des Reaktors unter Druck aufgegeben, während aus dem unter dem Reaktor befindlichen Abscheider eine kleine Menge von durchweg 10% des theoret benötigen Wasserstoffgases als Abgas entspannt wurde.

Bei der Durchführung der Hydrierung in der Flutphase wurde der gleiche Reaktor benutzt wie bei der Rieselphasehydrierung. Wasserstoff und zu hydrierende Komponente (verdünnt mit dem Hydrierprodukt als Verdünnungsmittel) wurden am unteren Ende des Reaktors unter Druck eingeführt. Am oberen Ende des Reaktors befand sich der Abscheider zur Trennung von Gas- und Flüssigphase, aus dem bei dem Abscheider der Rieselphase die Produkte entspannt wurden.

### Katalysatoren

Als Katalysatoren wurden Palladium, Ruthenium und Nickel oder deren Kombinationen eingesetzt, die auf Lithium-Aluminium-Spinell als Katalysatorträger aufgebracht worden waren.

Die Herstellung des Katalysator-Trägers sei beispielhaft geschildert:

2.86 Liter kugelförmiges $\gamma$-Aluminiumoxid mit 1—3 mm bzw. 4—6 mm Durchmesser und einer inneren Oberfläche von ca. 250 m²/g wurden mit Liter wäßriger Lösung bei 30°C getränkt, in die nacheinander 296 g Ameisensäure und 233 g 54-%ige wäßrige Lithiumhydroxidlösung gegeben worden waren. Das getränkte Aluminiumoxid wurde bei 15°C im Vakuum getrocknet, mit der gleichen Lösung nochmals getränkt und wieder bei 150°C im Vakuum getrocknet. Der Träger wurde anschließend 6 Std. bei 1050°C zum Lithium-Aluminium-Spinell geglüht, was durch eine Röntgenaufnahme bestätigt wurde (60% Spinell). Der fertige Träger hatte eine innere Oberfläche von ca. 30 m²/g und eine mittlere Porenweite von ca. 700 Å.

Das Aufbringen der Edelmetalle erfolgte in der herkömmlichen Art durch Tränken mit wäßrigen Lösungen der Edelmetallsalze, Reduktion, Waschen (frei von Anionen) und Trocknen. Auf diese Weise wurden die Katalysatoren (= Katalysator + Katalysatorträger) hergestellt, welche die folgenden Mengen an Edelmetall aufgebracht enthielten:

| Katalysator-Nr. | % Edelmetall |
|---|---|
| 1 | 1 % Pd |
| 2 | 1 % Ru |
| 3 | 0,5 % Pd 0,5 % Ru |
| 4 | 5 % Ni |

#### Hydrierbeispiel 1

Tetrahydrophthalsäureanhydrid wurde, verdünnt mit Hexahydrophthalsäureanhydrid (1:10), mit einer Raum-Zeit-Ausbeute von 1 kg Hexahydrophthalsäureanhydrid/l Katalysator und Stunde in der beschriebenen Hydrierapparatur über 3 l Katalysator Nr. 1 in der Rieselphase bei 100 bar und 105°C kontinuierlich hydriert. Aus dem abscheider wurden 400 Nl/h Abgas (Nl = Liter unter Normalbedingungen) entspannt. Die Ausbeute an Hexahydrophthalsäureanhydrid betrug über 99%. Hexahydrophthalsäure und Hexahydrophthalid als Nebenprodukte werden praktisch nicht gebildet.

#### Hydrierbeispiel 2

Benutzte man Katalysator Nr. 2 anstelle von Katalysator Nr. 1, so wurden unter den gleichen Bedingungen wie im Beispiel 1 99,5% Ausbeute an Hexahydrophthalsäureanhydrid erhalten, als Nebenprodukt trat Hexahydrophthalid auf.

#### Hydrierbeispiel 3

Tetrahydrophthalsäureanhydrid wurde unverdünnt mit einer Raum-Zeit-Ausbeute von 800 g/l Katalysator und Stunde in der Rieselphase über 3 l Katalysator Nr. 3 kontinuierlich bei 120 bar und 95°C hydriert. Aus dem Abscheider wurden 400 Nl Abgas/h entspannt. Die Ausbeute an Hexahydrophthalsäureanhydrid betrug 99,2%. Als Nebenprodukt wurde etwas Hexahydrophthalid gebildet.

#### Hydrierbeispiel 4

Tetrahydrophthalsäureanhydrid wurde mit Hexahydrophthalsäureanhydrid im Verhaltnis 1:6 verdünnt. Mit einer Raum-Zeit-Ausbeute von 1,0 kg Hexahydrophthalsäureanhydrid pro l Katalysator und Stunde wurde in der beschriebenen Apparatur in der Flutphase über 3 l Katalysator Nr. 4 bei 120 bar und 100°C kontinuierlich hydriert. Aus dem Abscheider wurden ca. 800 Nl/h Abgas entspannt. Die Ausbeute an Hexahydrophthalsäureanhydrid betrug 99%. Hexahydrophthalid und Hexahydrophthalsäure waren die einzigen Nebenprodukte.

### Patentanspruch

Verfahren zur katalytischen, kontinuierlichen Hydrierung von Δ4-Tetrahydrophthalsäureanhydrid zu Hexahydrophthalsäureanhydrid in Gegenwart mindestens eines im Festbett angeordneten Hydrierungskatalysators in flüssiger Phase bei Temperaturen von 70—150°C und 30—200 bar in der Riesel- oder Flutphase, dadurch gekennzeichnet, daß man die Hydrierung in Gegenwart eines im Festbett angeordneten Katalysators aus der Reihe Palladium, Ruthenium oder Nickel oder deren Kombinationen durchführt, der Katalysator auf einem Träger aus Aluminiumoxide, das mindestens zu 20% in Lithium-Aluminium-Spinell umgewandelt worden ist, aufgebracht ist, und die zu hydrierende flüssige Phase aus einem Gemisch von Δ4-Tetrahydrophthalsäureanhydrid und Hexahydrophthalsäureanhydrid im Gewichtsverhältnis 1:1 bis 1:100 besteht.

### Revendication

Procédé pour l'hydrogénation catalytique continue de l'anhydride Δ4-tétrahydrophtalique en anhydride hexahydrophtalique en présence d'au moins un catalyseur d'hydrogénation disposé en lit fixe, en phase liquide, à des températures de 70 à 150°C et des pressions de 30 à 200 bars, en phase ruisselante ou noyée, ce procédé se caractérisant en ce que l'on effectue l'hydrogénation en présence d'un catalyseur de la série du palladium, du ruthénium ou du nickel ou de leurs combinaisons disposé en lit fixe, le catalyseur étant appliqué sur un support qui consiste en alumine qui a été convertie en proportions d'au moins 20% en spinelle de lithium-aluminium, et en ce que la phase liquide à hydrogéner consiste en um mélange d'anhydride Δ4-tétrahydrophtalique et d'anhydride hexahydrophtalique dans des proportions relatives en poids de 1:1 à 1:100.

### Claim

Process for the catalytic, continuous hydrogenation in liquid phase of Δ⁴-tetrahydrophthalic acid anhydride to hexahydrophthalic acid anhydride, at temperatures from 70°C to 150°C and at from 30 to 200 bar, in the trickle or flood phase, in the presence of at least one hydrogenation catalyst arranged in a fixed bed, characterised in that the hydrogenation is carried out in the presence of a catalyst from the series comprising palladium, ruthenium or nickel or combinations thereof, arranged in a fixed bed, the catalyst is applied to a carrier of aluminium oxide of which at least 20% have been converted into lithium aluminium spinel and the liquid phase to be hydrogenated consists of a mixture of $\Delta^4$-tetrahydrophthalic acid anhydride and hexahydrophthalic acid anhydride in a weight ratio of 1:1 to 1:100.